# EUROPEAN PATENT APPLICATION

(11) **EP 4 492 055 A1**
(43) Date of publication of application: **15.01.2025**
(21) Application number: 23020344.0
(22) Date of filing: 12.07.2023
(51) Int. Cl.: G01N 33/00, B01F 23/10

(54) **METHOD AND APPARATUS FOR PROVIDING A CALIBRATION GAS BY MEANS OF DILUTION**

(71) Applicant: Linde GmbH, 82049 Pullach (DE)
(72) Inventor: Adam, Peter, 82049 Pullach (DE)
(74) Representative: Reuß, Stephanie

(57) **Abstract**

The invention relates to a method and an apparatus (100) for providing a calibration gas by means of dilution, wherein one or more first gas flows (a, b), each with a respective first flow rate (115), are mixed with a second gas flow (c) with a second flow rate (125), in order to obtain a diluted gas flow (d). The pressure of the diluted gas flow is controlled (160) to a desired pressure in order to obtain a calibration gas flow (d), and the calibration gas of the calibration gas flow (d) is provided to at least one application (170).

## Description

The present invention relates to a method and an apparatus for providing a calibration gas mixture by means of dilution, e.g., for different applications

### Background

Dynamic dilution methods can be used to dilute gases from sources with higher concentration to lower concentration ranges for various applications. These can be applied for calibrating and testing of analysers. Typical ways of preparing (calibration) gases turned out to have different disadvantages. It is therefore an object of the present invention to provide an improved way of providing a calibration gas.

### Disclosure of the invention

This object is achieved by providing a method and an apparatus for providing a calibration gas with the features of the independent claims. Embodiments of the invention are the subject of the dependent claims and of the description that follows.

The invention relates to providing a calibration gas by means of dilution, in particular, by means of dynamic dilution or high-pressure dynamic dilution. Note that a calibration gas can - and typically will - comprise a mixture of different pure gases. In the following, the term gas shall comprise a particular pure gas as well as a mixture of two or more pure gases, unless otherwise specified.

Dynamic dilution methods can be used to dilute gases from sources with higher concentration to lower concentration ranges for various applications. These diluted gases can be applied for calibrating and testing of analysers. Dilution of gases using mass flow controllers can be used for analyser calibration at or close to ambient pressure. Dynamic dilution allows the generation of gases which can be employed either to calibrate the analysers or to validate gases contained in high pressure cylinders. Dynamic dilution can be achieved by using mass flow controllers (MFCs) due to their ease of use and good level of reliability. Mass flow controller sensors can be based, for example, on heat exchange equations, therefore the response of these instruments is sensitive to the nature of the flowing gas. The different response can be considered by means of a so-called gas correction factor (GCF), which can be computed when the gas properties are known.

Such standard ways of preparing (calibration) gases, however, turned out to have different disadvantages. The production of (calibration) gases typically is a multi-stage process that has to run through different production locations and/or systems. A first state is the pre-treatment of cylinders (reservoir for the pre-mixed gas or the reservoir to receive the calibration gas), mainly the passivation of gas cylinders in laboratories using different raw materials. The cylinder is disconnected from the system after filling with the raw materials (pre-mixes). A further stage is the static (in turn, e.g., multi-stage) cylinder filling using gravimetric or manometric filling rigs, for example. The cylinder is disconnected once the filling process is finalized. A further stage is that the (calibration) gas is analysed. Beforehand, the analyser is typically calibrated with a calibration standard.

In addition, lower amount fractions (e.g., ppb-level, 10⁻⁹ mol/mol) of reactive components (e.g., HF, CH2O, ...) cannot be filled with static methods like gravimetry or manometry because of the instability of these molecules due the reaction of the components with the inner cylinder wall. In addition, for the preparation of calibration gases with amount fractions in the ppm-level (10⁻⁶ mol/mol) or below pre-mixtures have to be used.

At least parts of these disadvantages can be overcome with the method and apparatus for providing a calibration gas by means of (dynamic) dilution, provided within the present invention. In the following, the method and the apparatus will be described in combination.

One or more first gas flows are provided. Each of the first gas flows, preferably, comprises a pre-mixed gas. Each of these first gas flows has respective first flow rate; it is noted that two or more first flow rates can differ from each other. Each of the first gases can be provided via a respective first gas reservoir (or gas cylinder), e.g. a gas reservoir comprising a pre-mixed gas. If two or more first gas reservoirs are used, these can comprise different first gases or pre-mixed gases. The one or more first gases are the ones to be diluted. The apparatus is configured to be connected to these one or more first gas reservoirs, e.g., via terminals. Note that corresponding valves and the like can be provided.

Further, the apparatus comprises one or more first mass flow controllers, e.g., of the type mentioned above. Each of them is configured to be supplied from a respective one of the first gas reservoirs and each of them is configured to provide the first gas flow, each with a respective first flow rate.

Further, a second gas flow with a second flow rate is provided. The second gas, preferably, comprises a zero or balance gas like nitrogen, helium, argon, hydrogen. The second gas is the one used to dilute the first gas(es). The second gases can be provided via a respective second gas reservoir (or gas cylinder), e.g. a gas reservoir comprising a zero or balance gas. The apparatus is configured to be connected to such second gas reservoir, in order to provide the second gas flow. Note that corresponding valves and the like can be provided.

Further, the apparatus comprises a second mass flow controller, e.g., of the type mentioned above, which is configured to be supplied from the second gas reservoir and is configured to provide the second gas flow, with the second flow rate.

The one or more first gas flows and the second gas flow are then mixed, in order to obtain a diluted gas flow. The apparatus can be configured to combine respective gas flows. Further, the apparatus can comprises a mixing device or homogenise the diluted gas flow. The one or each of the more first gas flow rates and the second gas flow rate can be controlled according to a desired dilution level of the dilution gas. Preferably, these flow rates are dynamically varied, if the desired dilution level varies over time (dynamic dilution).

The mass flow controllers can be configured to allow respective flow rates. For example, the one or one of the more first mass flow controllers can be configured to be controlled to provide a flow rate of up to 1l/min, or of up to 10l/min. If two first mass flow controllers are used, one of them can be configured to be controlled to provide a flow rate of up to 1l/min and the other one of up to 10l/min, for example. The second mass flow controller can be configured to be controlled to provide a flow rate of up to at least 100l/min. This allows a broad range of dilution. It is noted that the final dilution of the dilution gas does not only depend on the different flow rates but also on a dilution of the first gas(es). As mentioned above, these first gases can be pre-mixed, which includes dilution.

Further, a pressure of the diluted gas flow is controlled to a desired pressure in order to obtain a calibration gas flow. The apparatus comprises a back pressure regulator, configured to control the pressure of the diluted gas flow in order to obtain the desired pressure of the diluted gas flow. In this way, the pressure of the diluted gas can be adjusted according to needs. In particular, the pressure of the diluted gas flow can also be varied over time or dynamically changed, according to needs.

Further, the calibration gas of the calibration gas flow is then provided to at least one application. The apparatus is configured to be connected to these one or more application devices or reservoirs, in order to provide these with the calibration gas. The at least one application comprises, for example, one or more of the following applications: calibrating an analyser or a spectrometer using the calibration gas; and filling one or more target reservoirs with the calibration gas. Such analyser or spectrometer or target reservoirs can be connected to the respective third terminal.

Controlling the pressure of the diluted gas flow, using the back pressure regulator, allows providing the calibration gas with much higher pressure than can be done by standard ways. The one or at least one of the more first gas flows can be provided with a pressure of at least 10 bar, 50 bar, or 100 bar or at least 150 bar or even at least 200 bar. Corresponding first gas reservoirs can be used here. Also, the second gas flow can be provided with a pressure of at least 100 bar or at least 150 bar or even at least 200 bar. A corresponding second gas reservoirs can be used here. The back pressure regulator allows controlling the initial high pressure of, e.g., 100 bar or more, down to low pressures of, e.g., 1 to 10 bar, if desired. This allows, for example, calibrating the analyser or spectrometer in a wide range, e.g., from 1 bar to 100 bar or higher.

With the method and apparatus described herein, the entire preparation of calibration gases (reservoir or cylinder pre-treatment, cylinder filling and analysis) can be done with a single device (apparatus without changing locations or systems. The dynamic volumetric method enables the production of stable calibration gases at the ppb-level.

By using suitable dilution levels in the range of 1/1.000.000, for example, the calibration gases can be produced even without pre-mixes. The calibration gases are then prepared from pure gases. This reduces the number of gas cylinders and gas mixtures needed to prepare the calibration gas.

Using the dynamic volumetric and high-pressure analysis system described above allows the gas cylinders be pre-treated (drying and passivation of inner cylinder surface) before filling. Dynamic volumetric filling up to the working pressure of the gas cylinder (mostly 200 bar) is enabled by use of a back pressure regulator, which ensures a constant gas dilution during the complete filling process. This ensures that the calibration gas is homogeneous and has a low blending tolerance.

The calibration gas can be analysed at the same time (real time analysis). The spectrometer can detect most components, only monatomic gases cannot be determined. The simultaneous filling and analysis of the gas mixture results in a reduction in the working time required for mixture production. Both processes can take place at the same place without having to move the target cylinder.

The high-pressure dynamic filling process allows to produce ppb-mixtures of reactive components like H2S, NH3, HF and the like. The process can also be used for batch filling. In this case, the technique achieves very good batch homogeneity in terms of gas mixture composition. The process can also be cascaded to fill several target reservoirs or cylinders at the same time (batch filling). The prerequisite for this is only a uniform volume of the target cylinders (e.g., 10 liters). The process is also suitable for dosing liquids which then evaporate in the target cylinder. The premix (or pure gas) and the residual gas can also be dosed sequentially. This allows higher levels of dilution to be achieved.

Further advantages and embodiments of the invention will become apparent from the description and the appended figure.

It should be noted that the previously mentioned features and the features to be further described in the following are usable not only in the respectively indicated combination, but also in further combinations or taken alone, without departing from the scope of the present invention.

Short description of the figures
- Fig. 1: illustrates a flow diagram for explaining an embodiment of the invention; and
- Fig. 2: illustrates a method according to embodiment of the invention.

### Detailed description of the figures

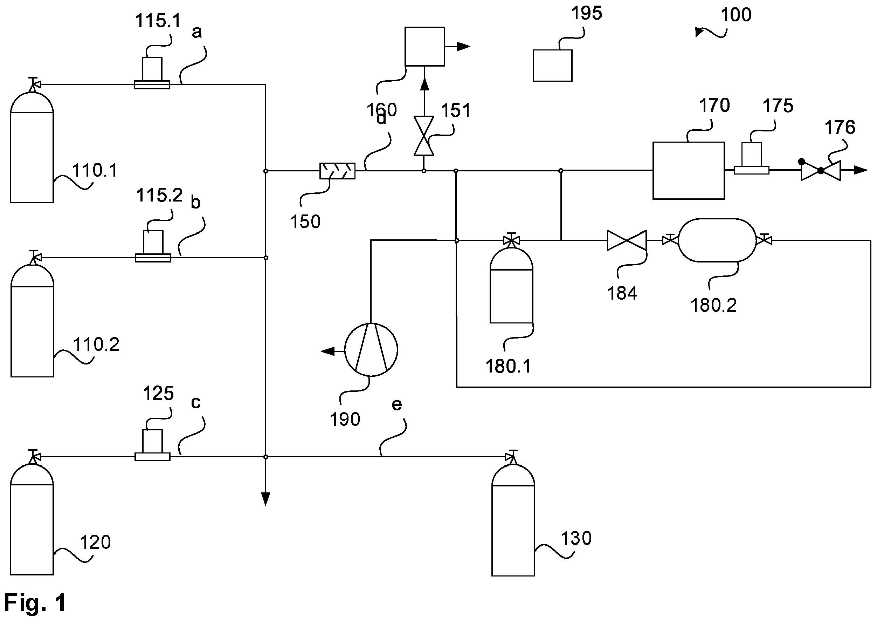

Fig. 1 schematically illustrates an apparatus 100 according to an embodiment of the invention, by means of a flow diagram, also illustrating a corresponding method of providing a calibration gas.

The apparatus 100 is configured to be connected to a first gas reservoir 110.1, e.g., via a respective terminal. The first gas reservoir 110.1 can be a 10- or 40-liter aluminum cylinder containing a premix, with a working pressure of 200 bar, for example. In Fig. 1, the apparatus 100 is connected to the first gas reservoir 110.1. The apparatus 100, thus, can receive a first gas supplied from the first reservoir 110.1. Further, the apparatus 100 comprises a first mass flow controller 115.1, configured to be supplied from the first gas reservoir 110 and configured to provide a first gas flow a, at first flow rate of, e.g., up to 1l/min.

The apparatus 100 is further configured to be connected to another first gas reservoir 110.2, e.g., via a respective terminal. The first gas reservoir 110.1 dan be a 10- or 40-liter aluminum cylinder containing a premix, with a working pressure of 200 bar, for example. In Fig. 1, the apparatus 100 is connected to the first gas reservoir 110.2. The apparatus 100, thus, can receive another first gas, supplied from the first reservoir 110. Further, the apparatus 100 comprises another first mass flow controller 115.2, configured to be supplied from the first gas reservoir 110.2 and configured to provide another first gas flow b, at a first flow rate of, e.g., up to 10l/min.

The apparatus 100 is further configured to be connected to a second gas reservoir 120, e.g., via a respective terminal. The second gas reservoir 120 can be a 50-liter cylinder containing a zero or balance gas like H2 N6.0 (or He or Ar), for example, at a filling pressure of 200 bar. Hydrogen, Argon or Helium can be used to dilute the premixes.

Note that the reservoir does not need to be a local cylinder but can also be a remote reservoir or other way to provide such second gas. In Fig. 1, the apparatus 100 is connected to the second gas reservoir 120. The apparatus 100, thus, can receive a second gas, supplied from the second reservoir 120. Further, the apparatus 100 comprises a second mass flow controller 125, configured to be supplied from the second gas reservoir 120 and configured to provide a second gas flow c, at a second flow rate of, e.g., up to 100l/min.

The apparatus 100 can further comprise valves and the like in order to provide the second gas flow c as desired. Note that pipes or lines for connection the individual components are not explicitly referred to.

The apparatus 100 is configured to combine or mix the first gas flows a, b and the second gas flow c, in order to provide a diluted gas flow d. The dilution depends on the flow rates chosen for the mass flow controllers. Preferably, the apparatus 100 can also comprise a mixing device or homogenization device 150, which is configured to homogenize diluted gas flow d.

The apparatus 100 is, by means of example, further configured to be connected to a reservoir 130, e.g., via a respective terminal, in order to provide a purge gas flow e. The gas reservoir 130 can be a 50-liter cylinder containing a purge gas like H2 N6.0 (or He or Ar), for example, at a filling pressure of 200 bar. Hydrogen, Argon or Helium can be used to purge the entire apparatus and its pipes. The apparatus 100 can further comprise valves and the like in order to provide the purge gas flow e as desired. Note that pipes or lines for connection the individual components are not explicitly referred to.

The apparatus 100 further comprises a back pressure regulator 160, configured to control a pressure of the diluted gas flow d in order to obtain a desired pressure of the diluted gas flow d. By controlling the back pressure regulator 160, pressure of the diluted gas flow d can be released such that a desired pressure is achieved. The diluted (and preferably homogenized) gas, preferably also after pressure regulation, can then be called the calibration gas to be provided.

Further, the apparatus 100 is configured to be connected to a gas analyser, for example, a spectrometer 170, e.g., via a terminal. A mass flow controller 175 can be provided after the spectrometer 170, in order to control the flow rate of the diluted or calibration gas flow through the spectrometer 170. The mass flow controller 175 can be configured to provide a flow rate of, e.g., up to 10l/min. A check valve 176 can be provided to exhaust gas.

The spectrometer can be, for example, a Raman spectrometer, in particular, a high pressure Raman spectrometer, configured for pressures of, e.g., up to 500 bar or 700 bar or more. The spectrometer can be used to analyse the component(s) in accordance with ISO 14687. Note that other types of spectrometers or analysers can basically be also used.

Further, the apparatus 100 is configured to be connected to a target reservoir 180.1 and a target reservoir 180.2, e.g., via terminals. Target reservoir 180.1 can be, for example, a 10-liter aluminum cylinder, with a working pressure of 200 bar. Target reservoir 180.2 can be, for example, a 0.5-liter sample cylinder, with working pressure at more than 300 bar.

The target reservoirs can, thus, be filled with the calibration gas such that the calibration gas can be used at another location and application. It is noted that these two target reservoirs are examples only; more or less than two reservoirs and also different types of reservoirs could be used, for example.

In addition, the apparatus 100 can further comprise valves and the like in order to provide the diluted or calibration gas flow d to the spectrometer 170, and the target reservoirs 180.1, 1802. Note that pipes or lines for connection the individual components are not explicitly referred to.

In addition, the apparatus 100 can further a pump or vacuum pump 190. This pump 109 can be used to evacuate the target reservoirs and pipes downstream of the mixing device 150.

In addition, the apparatus 100 can further comprise a processing device 195, configured to control the individual mass flow controllers and the back flow regulator in order to (dynamically) achieve a desired dilution, desired flow rates and a desired pressure of the diluted or calibration gas. Depending on the types of valves, the processing device 195, for example, can also be configured to control valves. Note that measuring devices for measuring flow and/or pressure can be used in the apparatus but are not explicitly mentioned here.

It is noted that the apparatus 100 can comprise further pipes, valves and the like in order to achieve the desired flows. Such other components are not illustrated for the sake of better understanding.

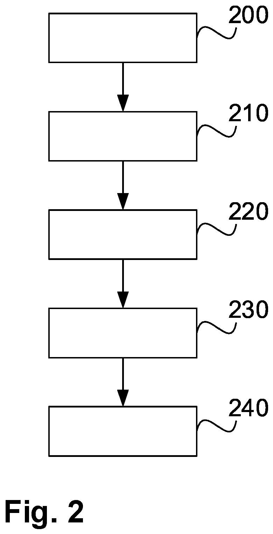

Fig. 2 briefly illustrates a method according to provide a calibration gas in an embodiment. In step 200, one or more first gas flows, each with a respective first flow rate, are provided; each of the first gas flows, preferably, comprising a pre-mixed gas. Note that multiple first flow rates can be different from each other.

In a step 210, a second gas flow with a second flow rate is provided, the second gas, preferably, comprising a zero or balance gas. In a step 220, the one or more first gas flows and the second gas flow are mixed, in order to obtain a diluted gas flow. In step 230, a pressure of the diluted gas flow is controlled to a desired pressure in order to obtain a calibration gas flow. In step 240, the calibration gas of the calibration gas flow is provided to at least one application, e.g., the spectrometer or filling a target reservoir.

In the following, a specific example of operating the apparatus, i.e., a process of dynamic volumetric filling of a target reservoir (or target cylinder) will be described.

At the beginning, starting parameters are present. One target cylinder, e.g., target reservoir 180.1 or 180.2 of above, can be used; it can have been evacuated, e.g., to 0,1 mbar (abs.) before. Further, one first gas reservoir, e.g., first gas reservoir 110.1 can be used, providing a first gas (premix). Further, the second gas, a zero gas, can be hydrogen. Argon (Ar) can be used as purge gas.

An Argon spectrum (baseline spectrum) can be obtained. First, a Helium spectrum can be carried out with the spectrometer at a targeted filling pressure of the target cylinder (5e.g., 0 bar). With a flow rate of 1l/min and a pressure rate of 50 bar, the Helium background spectrum can be taken with a measuring time of 1 minute.

The target cylinder can then be filed, with simultaneous analysis. The cylinder valves of first reservoir 110.1 and second reservoir 120 can be open as well, all other valves can be closed. The spectrometer 170 can now be used to analyse during the filling process in 1-minute intervals.

Different pressure and/or flow rate values can be used; also, more than one first gas reservoirs and/or more than one target reservoirs can be used. Also, only filling or only analysing can be done.

## Claims

1. A method for providing a calibration gas by means of dilution, comprising the following steps:
Providing (200) one or more first gas flows (a, b), each with a respective first flow rate, each of the first gas flows, preferably, comprising a pre-mixed gas;
Providing (210) a second gas flow (c) with a second flow rate, the second gas, preferably, comprising a zero or balance gas;
Mixing (220) the one or more first gas flows and the second gas flow, in order to obtain a diluted gas flow (d);
Controlling (230) a pressure of the diluted gas flow to a desired pressure in order to obtain a calibration gas flow (d); and
Providing (240) the calibration gas of the calibration gas flow (d) to at least one application.

2. The method of claim 1, wherein the at least one application comprises one or more of the following applications:
calibrating an analyser or a spectrometer (170) using the calibration gas;
filling one or more target reservoirs (180.1, 180.2) with the calibration gas.

3. The method of claim 1 or 2, wherein the diluted gas flow (d) is provided by means of dynamic dilution.

4. The method of any one of the preceding claims, wherein the one or at least one of the more first gas flows (a, b) is provided with a pressure of at least 10 bar, or at least 50 bar or at least 100 bar or at least 150 bar or at least 200 bar.

5. The method of any one of the preceding claims, wherein a dilution level of the calibration gas is in the range of 1/1.000.000, or less, preferably in the range of 1/1.000.000.000.

6. The method of any one of the preceding claims, wherein the one or each of the more first gas flow rates and the second gas flow rate are controlled according to a desired dilution level of the dilution gas.

7. The method of any one of the preceding claims, wherein the zero or balance gas is one of the following gases or mixtures of them: nitrogen, helium, argon, hydrogen, methane, natural-gas, oxygen, or other permanent gases

8. An apparatus (100) for providing a calibration gas by means of dilution, configured to be connected to one or more first gas reservoirs (110.1, 110.2), configured to be connected to a second gas reservoir (120), and configured to be connected to on e or more application devices (170) or reservoirs (180.1, 180.2), wherein the apparatus (110) comprises:
one or more first mass flow controllers (115.1, 115.2), each configured to be supplied from a respective one of the first gas reservoirs (110.1, 110.2) and configured to provide a first gas flow (a, b), each with a respective first flow rate;
a second mass flow controller (125), configured to be supplied from the second gas reservoir (120) and configured to provide a second gas flow with a second flow rate;
wherein the apparatus (100) is configured to receive and mix the one or more first gas flows (a, b) and the second gas flow (c), in order to provide a diluted gas flow (d);
wherein the apparatus further comprises a back pressure regulator (160), configured to control a pressure of the diluted gas flow (d) in order to obtain a desired pressure of the diluted gas flow (d); and
wherein the apparatus (100) is further configured to provide calibration gas of the calibration gas flow to the one or more application devices (170) or reservoirs (180.1, 180.2).

9. The apparatus (100) of claim 8, wherein the one or one of the more first mass flow controller (115.1, 115.2) is configured to be controlled to provide a flow rate of up to 1l/min, or of up to 10l/min.

10. The apparatus (100) of claim 8 or 9, wherein the second mass flow controller (125) is configured to be controlled to provide a flow rate of up to at least 100l/min.

11. The apparatus (100) of any one of claims 8 to 10, configured to be supplied with a gas pressure of at least up to 200 bar.

12. The apparatus (100) of any one of claims 8 to 11, configured to perform the method of any one of claims 1 to 7.
